## Europäisches Patentamt
## European Patent Office
## Office européen des brevets

(11) Publication number: **0 013 830**
**B1**

(12) **EUROPEAN PATENT SPECIFICATION**

(45) Date of publication of patent specification: **26.01.83**

(21) Application number: **79303035.4**

(22) Date of filing: **21.12.79**

(51) Int. Cl.³: **C 12 N 15/00,**
**C 12 P 21/00, C 12 P 19/34**

(54) Stable high copy number plasmids, their formation and use in protein production.

(30) Priority: **26.12.78 US 972705**

(43) Date of publication of application:
**06.08.80 Bulletin 80/16**

(45) Publication of the grant of the patent:
**26.01.83 Bulletin 83/4**

(84) Designated Contracting States:
**AT BE CH DE FR GB IT LU NL SE**

(56) References cited:
**EP - A - 0 003 062**

**CHEMICAL ABSTRACTS, vol. 90, no. 1, January 1, 1979, page 295, no. 3001v.
Columbus, Ohio, USA
B. E. UHLIN et al. "A runaway-replication mutant of plasmid R1d-rd-19: temperature-dependent loss of copy number control".**

(73) Proprietor: **CETUS CORPORATION
600 Bancroft Way
Berkeley, California (US)**

(72) Inventor: **Gelfand, David Harow
555 Pierce Street Apartment 505
Albany, California (US)**

(74) Representative: **Bizley, Richard Edward et al,
BOULT, WADE & TENNANT 27 Furnival street
London EC4A 1PQ (GB)**

Courier Press, Leamington Spa, England

(56) References cited:

CHEMICAL ABSTRACTS, vol. 90, no. 11, March 12, 1979, page 292, no. 83515d.
Columbus, Ohio, USA
D. H. GELFAND et al. "Isolation and characterization of a Co1E1-derived plasmid copy-number mutant"

CHEMICAL ABSTRACTS, vol. 89, no. 7, August 14, 1978, page 290, no. 56286c
Columbus, Ohio, USA
P. H. O'FARRELL et al. "Regulated expression by readthrough translation from a plasmid-encoded beta-galactosidase".

CHEMICAL ABSTRACTS, vol. 91, no. 5, July 30, 1979, page 304, no. 35558u.
Columbus, Ohio, USA
T. OGURA et al. "Control of chromosomal replication: Copy mutants of minichromosomes in Escherichia coli".

CHEMICAL ABSTRACTS, vol. 89, no. 19, November 6, 1978, page 283, no. 159981e.
Columbus, Ohio, USA
P. M. ANDREOLI et al.: "Isolation and characterization of a Clo DF1-3::Tn901 plasmid mutant with thermosensitive control of DNA replication".

Stable high copy number plasmids, their formation and use in protein production

This invention relates to the field of molecular biology and, more particularly, is based on a technique for increasing the levels of expression of protein products encoded by heterologous DNA in cloning vehicles.

Genetic change can occur randomly as a result of mutations appearing in a gene. As a result of change in the gene, a corresponding change may occur in the protein which it encodes, changing the resultant properties of the organism. With the advent of recombination DNA techniques, such genetic changes may be made deliberately by the introduction of a known nucleotide sequence from one strain or species into another. The known nucleotide sequence may be selected to confer a desired property upon the strain or species into which it is introduced. When the modified strain or species proceeds with the normal replication process, it also then duplicates the inserted sequence.

Recombinant DNA techniques involve isolating a suitable piece of a DNA chain (a vector or cloning vehicle) and breaking or severing the two strands of DNA of a cloning vehicle at the desired location where the foreign DNA is to be inserted. To do this, particular types of proteins, called restriction enzymes, are typically used. Certain restriction enzymes will break the DNA at particular nucleotide sequences, although the break may not necessarily occur at the same point on the two intertwined DNA strands. If two different types of DNA are severed in a similar matter, the open ends will therefore be complementary and will, under suitable conditions, stick together with the complementary ends lying side by side. They may then be linked together enzymatically (with ligase). This makes it possible to recombine two DNA segments from any source into a single DNA molecule.

All DNA, whether from microbes or from complex plants or animals, consists of the same identical nucleotides. Thus, when a DNA fragment derived from a foreign source is spliced into the DNA of a given species, the replication system of the host reproduces the inserted segment along with the DNA of the original host.

Once the DNA vector or cloning vehicle has been isolated and the foreign piece inserted therein, the recombinant DNA is then placed into a suitable host organism. In order for the host organism to replicate the inserted DNA, it is necessary that the recombinant DNA be inserted into the host in such a way as to become part of its genetic system.

In connection with the bacterium *Escherichia coli*, two convenient types of cloning vehicles have been utilized. *E. coli* bacteria, in addition to the main DNA chain or chromosome, frequently have one or more independently replicating circular loops of DNA known as plasmids. Also, a certain type of virus known as a lambda bacteriophage (phage) is also capable of infecting *E. coli* and becoming part of its genetic system. Recombination DNA techniques have included the use of a variety of plasmids or phages as cloning vehicles. This involves the isolation of plasmids or phages from the bacteria, the breaking open of the isolated DNA by restriction enzymes, the insertion of a heterologous piece of DNA into the plasmid or phage, the restoration of the circular form of the plasmid or the phage structure, and the return of the plasmid or phage to the *E. coli* cell.

Once in the host, the heterologous DNA is not only replicated from generation to generation, but also will produce protein for which it is encoded. (This assumes the proper reading frame and prometers exist). The amount of protein produced by heterologous DNA as a result of recombination depends, of course, on the magnitude and efficiency of the fermentation or other process used to exploit protein production and replication of the modified host bacteria. Another factor involved in the amount of protein produced is the amount of efficiency of protein production in each bacterium. The proportion of materials produced by heterologous DNA to that produced by the host's own DNA is typically the same from cell to cell and from generation to generation.

In many types of bacteria, only one copy of a cloning vehicle typically exists per cell. That is, there is a single plasmid per cell per chromosome. Some plasmids, however, exist in higher copy number than one. For example, ColEl typically contains 10 to 20 plasmid copies per chromosome. In certain plasmids, it is possible to increase relative proportion of plasmid DNA in the cell by adding a protein synthesis inhibitor such as chloramphenicol or spectinomycin. Of course, this technique does not assist in the accumulation of protein because of the presence of inhibitor.

Protein production may be enhanced by the use of so-called super promoters which provide for extremely high levels of protein expression. Such a technique has limitations, however, in the maximum rate at which the cellular machinery can operate.

The present invention provides a DNA plasmid comprising a site for replication origin, a site for the initiation of readthrough expression, a section of heterologous DNA appended to said expression initiation site encoded and in proper reading frame to produce a desired protein, and altered repressor gene leading to high copy number replication, and said plasmid also having a configuration such that translocatable elements are disabled.

The invention further includes a method for increasing the capability of production of protein by heterologous DNA in a plasmid cloning vehicle comprising selecting mutants of the plasmid having altered repressor genes leading to high copy number replication and disabling translocatable elements in the plasmid.

Further included in the invention is a method for producing a desired protein which comprises introducing a plasmid which has been subjected to the above method into the genetic system of a host organism and permitting the organism to grow and phenotypically express its genotype.

The invention will now be described and illustrated further with reference to the accompanying drawings, wherein:

FIGURE 1 is a schematic diagram of a plasmid pBGP120;

FIGURE 2 illustrates a copy number mutant plasmid derived from the plasmid of FIGURE 1 wherein a deletion has occurred; and

FIGURE 3 is a schematic diagram of a form of plasmid constructed in accordance with the invention.

Very generally, the plasmid of the invention comprises a sequence of DNA containing a site 11 for replication origin, a site 13 for the initiation of readthrough expression, and a section 15 of heterologous DNA appended to the expression initiation site and encoded and in the proper reading frame to produce a desired protein. The sequence further has a disabling configuration which results in a plasmid of high copy number capability which is stable in that deletions are substantially avoided.

In constructing the plasmid of the invention, a plasmid is selected which is useful as a cloning vehicle. One such plasmid is shown and described in Journal of Bacteriology May 1978, Volume 134 No. 2 pages 645 to 654. This plasmid contains a functional portion (the *lac* promoter and *lac* operon) of an indigenous gene (the betagalactosidase gene) of the host bacteria, terminating in an EcoRI restriction site 14. This portion may be linked at the restriction site 14 to a heterologous gene orientated in the same orientation and having the same reading frame such that readthrough can occur from the indigenous gene portion into the heterologous gene in the same reading frame.

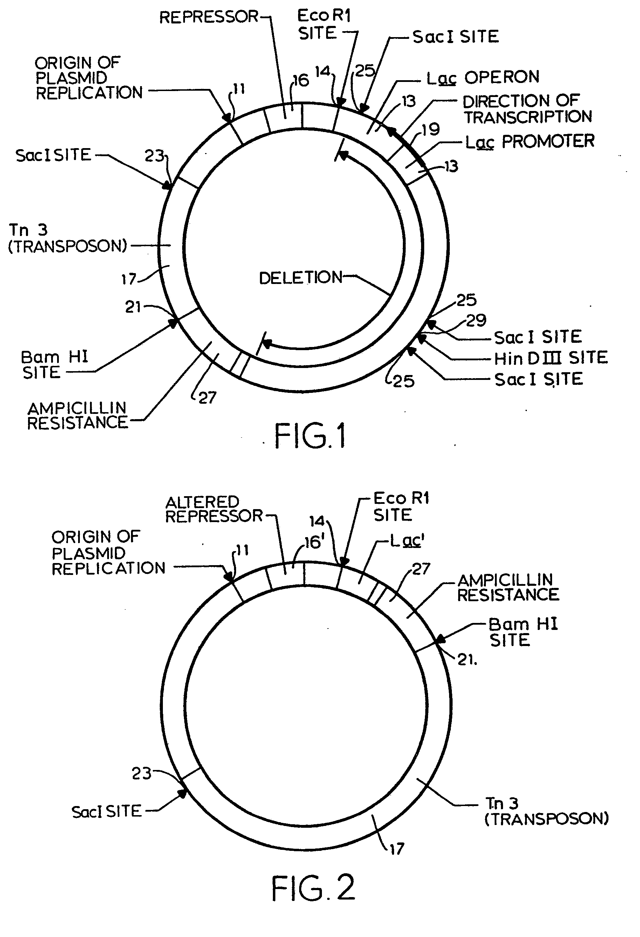

This plasmid (pBGP120) exists in high copy number levels, approximately 10 to 20 plasmids per cell chromosome. Plasmids capable of existing at even higher copy numbers are selected by typical prior art techniques. Such techniques include titrating levels of a cloned product produced by the plasmid DNA. In order to stimulate the presence of high copy number characteristics, it has been concluded that it is typically a repressor produced by the repressor gene 16 of the plasmid itself which keeps the copy number down, rather than some positive control which provides a stimulus that maintains an elevated copy number. The present invention is preferably applied to a plasmid where a negative control system of this type is present. In this connection, reference is made to the work on PSC 101 chimeras, Cabello, et al. (1976) Nature *259*; 285—290. Typically, several successive screening procedures may be required to select the high copy number mutants. In other words, it is important to the invention that the characteristic of high copy number is indigenous to the plasmid itself, and is not the result of any function uniquely possessed by a particular host bacteria. In any case, the selected plasmid has an altered repressor gene 16' (FIGURE 2) leading to high copy number, i.e. in excess of 10—20 per chromosome.

It has been discovered that plasmids thus selected, even though existing in high copy number, almost invariably undergo indesirable deletions of substantial portions of the plasmid (i.e. are unstable). In fact, loss of critical portions of the plasmid, such as the promoter which promotes translation of the inserted heterologous DNA, results in failure of the plasmid to produce the protein desired. The form of the high copy number plasmid is shown in FIGURE 2. It may be seen that with the *lac* promoter and much of the *lac* operon missing, DNA inserted at the EcoRI site 14 may be replicated, but transcription can no longer be controlled from the *lac* promoter.

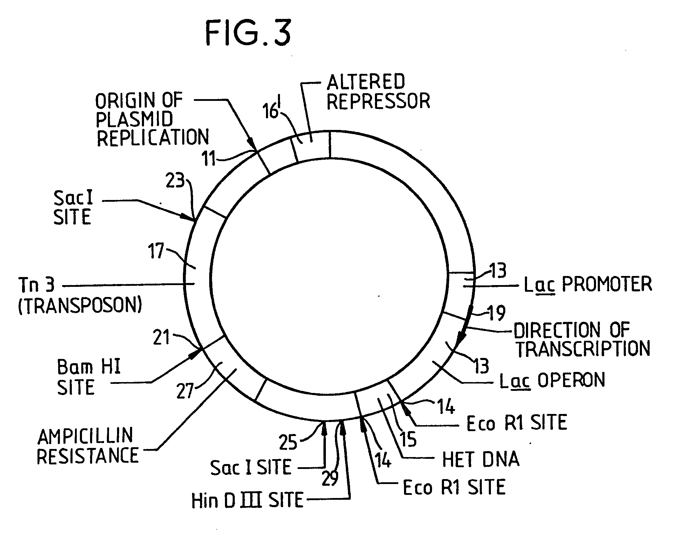

The direction of transcription of the *lac* promoter is shown in FIGURES 1 and 3 by the arrow 19. It will be noted in FIGURE 1 that the transcription of the Tn3 element is in the same direction as *lac*. Thus, the active *lac* promoter occasionally will give readthrough transcription into the Tn3 element 17. So, with the above model in mind, an approach can be utilized to generate stable, full-sized mutants. This approach is to turn the *lac* promoter and operon around so that it transcribes away from the Tn3 region.

Referring to FIGURE 1, it may be seen that the *lac* operon is bracketed by EcoR1 and HinDIII sites 14 and 29, respectively. The plasmid pBGP120 is cut by these two enzymes and fragments separated by size. The selected *lac* fragment is now mixed with HinDIII-EcoR1 bi-functional linkers. As is known, these linkers have an open HinDIII site at one end and an open EcoR1 site at the other. A certain percentage of the time, one linker will attach itself to each end of the *lac* fragment. They are ligated into place covalently. This fragment can now be reconnected to the other plasmid fragment in the opposite orientation. Transcription is now away from the Tn3 region. Thus, the plasmid is stable.

One further change is required to insert the heterologous DNA. It may be seen that the above-described reversal will leave two EcoR1 sites at each end (and two HinDIII sites as well). Unwanted EcoR1 sites (and HinDIII sites) may be removed by appropriate known techniques, such as partial digestion of the DNA followed by exonuclease treatment (Polisky et al; Proc. Nat'l.

Acad. Sci. *73*: 3900—3904). These plasmids may now be screened for high copy number mutants.

It is useful to have temperature-sensitive (Ts) copy number mutants. That is because high level production of some protein products of heterologous DNA may have a deleterious effect on the host cells. In temperature-sensitive copy number mutants, there is a maintenance of low copy number when the host cells are grown at a "permissive" temperature (usually relatively lower). When the product of the heterologous DNA is desired in quantity, the temperature is shifted (usually increased). At this non-permissive or restrictive temperature, the mutation manifests itself and the plasmid copy number increases, resulting in increased protein production from the plasmids.

Such Ts mutants are rare, but can be found for almost any type of mutant by appropriate selection. First the location of the copy number mutant is determined by fine-mapping with genetic recombination. Once the location is known, the spot is specifically mutagenized. This can be done by nuleotide alteration or by cutting out the region with restriction enzymes, strongly mutagenizing the fragment with some agents such as bisulfite, hydroxylamine or ultraviolet radiation, and replacing the fragment. The resultant plasmids are transformed into cells that overproduce *lac* repressor. These are then plated over Xgal at permissive temperatures. Blue regions are removed from the culture. Then, at elevated temperatures, new blue areas that appear are temperature-sensitive mutants which are picked.

These mutations are generally "recessive in trans.". That means, when a mutant and a normal plasmid are put in the same cell, the altered or "high copy number phenotype" is masked.

The deleted form of the copy number mutant can exist in the same cell as the normal copy number parental-type plasmid. (This is not true of the non-mutant because of the property of incompatability. In spite of the similar mode of replication, this property is not manifested with the deleted form.) When they are placed in the same cell, the copy number of both types of plasmid falls to normal (10—15). Thus, it can be concluded that a diffusable product — a repressor — normally maintains the low copy number, but is absent or inactive in mutant form.

However, it is useful to have a copy number mutant that is dominant in trans. This allows a more generalized approach in that non-copy number mutant plasmids with similar modes of replication can be grown to a high copy level in hosts carrying such a transdominant mutation.

A type of mutation that gives high copy number is one which makes a "bad" repressor, instead of none at all. Repressors are, typically, not single units, but are multimers of identical proteins. As such, when good and bad repressors are placed in the same cell, their subunits can mix, and none of the resulting repressors will be active. Thus, we have a mutation which is transdominant. Such a mutation can easily be obtained in a temperature-sensitive form, since a repressor has to be present for activity to exist at a permissive temperature. Such a mutation can be cloned into the plasmid.

It may be seen, therefore, that the invention provides an improved plasmid capable of existing and replicating in high copy number and of giving correct expression of protein. By high copy number, it is meant typically in excess of fifty plasmids per chromosome and preferably in the range of one hundred to five hundred or greater. The plasmid thus enables one to obtain very high level production of protein from heterologous DNA.

## Claims

1. A DNA plasmid comprising a site for replication origin, a site for the initiation of readthrough expression, a section of heterologous DNA appended to said expression initiation site encoded and in proper reading frame to produce a desired protein, and an altered repressor gene enabling the possibility of high copy number replication characterised in that said plasmid also has a disabling configuration obviating readthrough expression of elements which as a result of expression mediate rearrangement of translocatable elements.

2. A plasmid according to claim 1 further characterised in that said disabling configuration is provided by said expression initiation site and said heterologous DNA being oriented in a manner opposite to the orientation of any elements which as a result of expression mediate rearrangement of translocatable elements.

3. A plasmid according to claim 1 or claim 2 further characterised in that expression of said repressor gene produces defective repressor.

4. A plasmid according to claim 1 or claim 2 further characterised in that expression of said repressor gene produces no repressor.

5. A plasmid according to claim 1 or claim 2 further characterised in that said repressor gene is a high copy number mutant repressor gene which is temperature-sensitive and transdominant.

6. A plasmid according to any one of claims 1 to 4 further characterised in that said expression initiation site comprises a site for the initiation of translation and at least a substantial portion of a gene which is indigenous to a host species of bacteria whereby readthrough occurs from said indigenous gene portion into said heterologous DNA.

7. A method for increasing the capability of production of protein by heterologous DNA in a plasmid cloning vehicle comprising selecting mutants of the plasmid having altered repressor genes leading to the possibility of high copy number replication and characterised by disabling translocatable elements in the plasmid.

8. A method according to claim 7 further characterised in that the plasmid includes a *lac* promoter and *lac* operon and a Tn3 region expression of which latter mediates rearrangement of translocatable elements, said disabling being achieved by orienting the *lac* promoter and *lac* operon so that reading thereof occurs in direction opposite to the reading direction of the Tn3 region.

9. A method according to claim 8 further characterised in that the copy number mutants are temperature-sensitive copy number mutants.

10. A method according to claim 8 further characterised in that the copy number mutants selected are dominant in trans.

11. A method for producing a desired protein characterised by introducing a plasmid which has been subjected to a method according to any one of claims 8 to 10 into the genetic system of a host organism and permitting the organism to grow and phenotypically express its genotype.

## Revendications

1. Plasmide à ADN comprenant un site pour l'origine de réplication, un site pour l'initiation de l'expression de lecture, une section d'ADN hétérologue attenante audit site d'initiation d'expression codée et dans un cadre de lecture approprié pour produire une protéine désirée et un gène répresseur altéré établissant la possibilité d'une réplication à nombre d'exemplaires élevé, caractérisé en ce que ledit plasmide a également une configuration de mise hors service empêchant l'expression de lecture des éléments qui, par suite de l'expression, servent au réarrangement d'éléments susceptibles de translocation.

2. Plasmide selon la revendication 1, caractérisé de plus en ce que la configuration de mise hors service est apportée par ledit site d'initiation d'expression et en ce que ledit ADN hétérologue est orienté de façon opposée à l'orientation de tout élément qui, par suite de l'expression, sert au réarrangement des éléments susceptibles de translocation.

3. Plasmide selon l'une des revendications 1 ou 2, caractérisé de plus en ce que l'expression dudit gène répresseur produit un répresseur défectif.

4. Plasmide selon l'une des revendications 1 ou 2, caractérisé de plus en ce que l'expression dudit gène répresseur ne produit pas de répresseur.

5. Plasmide selon l'une des revendications 1 ou 2, caractérisé de plus en ce que ledit gène répresseur est un gène répresseur de mutant relatif à un nombre d'exemplaires élevé qui est sensible à la température et transdominant.

6. Plasmide selon l'une quelconque des revendications 1 à 4, caractérisé de plus en ce que ledit site d'initiation d'expression comprend un site pour l'initiation de la translation et au moins une portion importante d'un gène qui est indigène à une espèce hôte de bactérie, si bien que la lecture s'effectue à partir de ladite portion de gène indigène dans ledit ADN hétérologue.

7. Procédé pour accroître la capacité de production d'une protéine par l'ADN hétérologue dans un véhicule de clonage constitué d'un plasmide, qui comprend la sélection de mutants du plasmide ayant des gènes répresseurs altérés permettant la possibilité d'une réplication d'un nombre d'exemplaires élevé et caractérisé par la mise hors service d'éléments susceptibles de translocation dans le plasmide.

8. Procédé selon la revendication 7, caractérisé de plus en ce que le plasmide comprend un promoteur *lac* et un opéron *lac*, et une région Tn3 dont l'expression sert d'intermédiaire pour le réarrangement d'éléments susceptibles de translocation, ladite mise hors service étant obtenue par orientation du promoteur *lac* et de l'opéron *lac* de façon à ce que leur lecture se produise dans une direction opposée à la direction de lecture de la région Tn3.

9. Procédé selon la revendication 8, caractérisé de plus en ce que les mutants relatifs au nombre d'exemplaires sont des mutants relatifs au nombre d'exemplaires qui sont sensibles à la température.

10. Procédé selon la revendication 8, caractérisé de plus en ce que les mutants relatifs au nombre d'exemplaires choisis sont dominants en trans.

11. Procédé pour produire une protéine désirée, caractérisée par l'introduction d'un plasmide que l'on a soumis à un procédé selon l'une quelconque des revendications 8 à 10 dans le système génétique d'un organisme hôte et en permettant à l'organisme de se développer et d'effectuer l'expression phénotypique de son génotype.

## Patentansprüche

1. DNA-Plasmid enthaltend eine Stelle für den Replikationsbeginn, eine Stelle zur Initiierung des Ableseausdrucks (readthrough expression), einen Abschnitt von heterologer DNA, der an diese kodierte Stelle zur Ausdrucksinitiierung angehängt ist und den geeigneten Lesemodus zur Bildung eines gewünschten Proteins aufweist, und ein verändertes Repressorgen, das eine Replikation mit hoher Reproduktionszahl ermöglicht, dadurch gekennzeichnet, daß dieses Plasmid auch eine inaktivierende Konfiguration besitzt, die einen Ableseausdruck von Elementen verhindert, die als Ergebnis des Ausdrucks die Umlagerung von Translokationselementen vermitteln.

2. Plasmid nach Anspruch 1, dadurch gekennzeichnet, daß die inaktivierende Konfiguration durch die Stelle der Ausdrucksinitiierung vorgesehen wird und die heterologe DNA entgegengesetzt orientiert ist zur Orientierung von Elementen, die als Ergebnis des Ausdruckes die Umlagerung der Translokationselemente vermitteln.

3. Plasmid nach Anspruch 1 oder 2, dadurch gekennzeichnet, daß der Ausdruck des Repressorgens einen unvollkommenen Repressor bildet.

4. Plasmid nach Anspruch 1 oder 2, dadurch gekennzeichnet, daß der Ausdruck des Repressorgens keinen Repressor bildet.

5. Plasmid nach Anspruch 1 oder 2, dadurch gekennzeichnet, daß das Repressorgen ein Mutantenrepressorgen mit hoher Reproduktionszahl ist, das temperaturempfindlich und transdominant ist.

6. Plasmid nach einem der Ansprüche 1 bis 4, dadurch gekennzeichnet, daß die Stelle zur Initiierung des Ausdrucks eine Stelle zur Translationsinitiierung enthält und mindestens einen wesentlichen Teil eines Gens einer Wirtsbakterienspezies, wobei das Ablesen von dem Genteil in die heterologe DNA erfolgt.

7. Verfahren zur Steigerung der Fähigkeit zur Proteinerzeugung durch heterologe DNA in einem klonisierenden Plasmidträger durch Selektion von Mutanten des Plasmids mit veränderten Repressorgenen, die eine Replikation mit hoher Reproduktionszahl ermöglichen, dadurch gekennzeichnet, daß man Translokationselemente in dem Plasmid inaktiviert.

8. Verfahren nach Anspruch 7, dadurch gekennzeichnet, daß das Plasmid einen *Lac*-Promoter und ein *Lac*-Operon und einen Tn3-Region-Ausdruck enthält, von denen der letztere die Umlagerung von Translokationselementen vermittelt, wobei die Inaktivierung erreicht wird durch Orientierung des *Lac*-Promoters und *Lac*-Operons, so daß ihr Ablesen in einer Richtung erfolgt, die entgegengesetzt ist zur Ableserichtung der Tn3-Region.

9. Verfahren nach Anspruch 8, dadurch gekennzeichnet, daß die Reproduktionszahl-Mutanten temperaturempfindliche Reproduktionszahl-Mutanten sind.

10. Verfahren nach Anspruch 8, dadurch gekennzeichnet, daß die ausgewählten Reproduktionszahl-Mutanten transdominant sind.

11. Verfahren zur Herstellung eines gewünschten Proteins, dadurch gekennzeichnet, daß man ein Plasmid, das einem Verfahren nach einem der Ansprüche 8 bis 10 unterworfen wurde, in das genetische System eines Wirtsorganismus einbringt und dem Organismus erlaubt zu wachsen und phänotypisch seinen Genotypus auszudrücken.

0 013 830

REPRESSOR — Eco R1 SITE — SacI SITE

ORIGIN OF PLASMID REPLICATION — 11

16 14 25

Lac OPERON

DIRECTION OF TRANSCRIPTION

13

19

Lac PROMOTER

13

SacI SITE — 23

Tn 3 (TRANSPOSON) — 17

DELETION

25

29

SacI SITE

Hin D III SITE

SacI SITE

25

21

Bam HI SITE

AMPICILLIN RESISTANCE — 27

FIG.1

ALTERED REPRESSOR —

Eco R1 SITE

ORIGIN OF PLASMID REPLICATION — 11

14

16'

Lac'

27

AMPICILLIN RESISTANCE

Bam HI SITE

21.

23

SacI SITE

Tn 3 (TRANSPOSON)

17

FIG.2

1

# FIG.3

ORIGIN OF PLASMID REPLICATION 11

ALTERED REPRESSOR 16¹

SacI SITE 23

Tn 3 (TRANSPOSON) 17

Bam HI SITE 21

AMPICILLIN RESISTANCE 27

Sac I SITE 25

Hin D III SITE 29

Eco R1 SITE 14

HET DNA 15

Eco R1 SITE 14

Lac OPERON 13

DIRECTION OF TRANSCRIPTION 19

Lac PROMOTER 13